(19) Europäisches Patentamt
European Patent Office
Office européen des brevets

(11) Publication number: **0 119 701 A1**

(12) # EUROPEAN PATENT APPLICATION

(21) Application number: **84300653.7**

(22) Date of filing: **02.02.84**

(51) Int. Cl.³: **C 07 B 17/00**, C 07 D 213/61, C 07 C 17/26, C 07 C 121/60, C 07 C 120/00

(30) Priority: **18.02.83 GB 8304613**

(43) Date of publication of application: **26.09.84 Bulletin 84/39**

(84) Designated Contracting States: **CH DE FR GB IT LI NL**

(71) Applicant: **IMPERIAL CHEMICAL INDUSTRIES PLC, Imperial Chemical House Millbank, London SW1P 3JF (GB)**

(72) Inventor: **Milner, David John, 18 Eight Acre, Whitefield Manchester M25 7LW (GB)**

(74) Representative: **Houghton, Malcolm John et al, Imperial Chemical Industries PLC Legal Department: Patents PO Box 6, Welwyn Garden City Herts, AL7 1HD (GB)**

(54) **Grignard syntheses.**

(57) A compound (I):

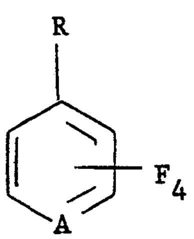

(I)

in which A is C–CN or N, R is alkyl, aralkyl, ($C_3$ or more) alkenyl, whose double bond is not attached to the carbon atom linked to the benzene or pyridine ring, or alkynyl, is prepared by treating a compound (II):

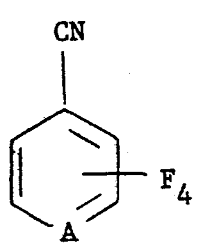

(II)

with 1 to 2 mols/mol of a Grignard reagent, RMgX.

Treatment of (I), in which A is C–CN, with further Grignard reagent gives a Grignard compound (III):

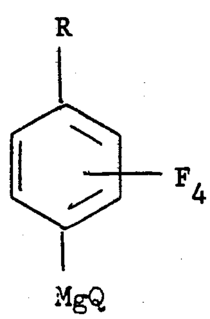

(III)

in which Q is CN or halo. Compound (III) can be obtained by a single stage process from tetrafluorotherephthalonitrile using more than 2 mols, preferably 3 mols, of Grignard reagent and may be used to form a compound (IV):

EP 0 119 701 A1

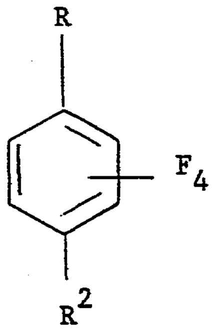

(IV)

in which $R^2$ is hydrogen, alkyl, alkenyl, hydroxy, carboxy, a carboxylic ester, formyl, hydroxyalkyl, alkoxyalkyl, or

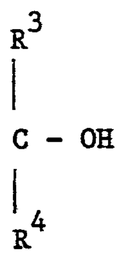

in which $R^3$ and $R^4$, which may be the same or different, are hydrogen or alkyl, by a Grignard reaction conventionally used to transform a Grignard compound $ArMgX^2$, in which Ar is aryl and $X^2$ is halo, into a compound $Ar-R^2$.

Compounds (I), (III) and (IV) are useful intermediates in the synthesis of pesticidal compounds.

GRIGNARD SYNTHESES

This invention relates to the preparation by Grignard syntheses of certain substituted tetrafluorobenzene and tetrafluoropyridine compounds which are useful chemical intermediates in the synthesis of pesticidal compounds.

More particularly, it relates to a process for preparing 1,2,4,5-tetrafluoro-3-cyano-6-hydrocarbylbenzenes from tetrafluoroterephthalonitrile and 2,3,5,6-tetrafluoro-4-hydrocarbylpyridines from the corresponding 4-cyanopyridine. It also relates to the transformation of the 1,2,4,5-tetrafluoro-3-cyano-6-hydrocarbylbenzenes into Grignard compounds, the use of the Grignard compounds to prepare various 3-substituted-1,2,4,5-tetrafluoro-6-hydrocarbylbenzenes, and the Grignard compounds themselves.

In one aspect of the invention there is provided a process for the preparation of a compound of the formula (I):

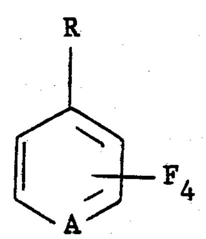

(I)

in which A is C-CN or N, R is alkyl, particularly (C1-4) alkyl, especially methyl and ethyl, aralkyl, especially benzyl or substituted benzyl, (C3 or more) alkenyl whose double bond is not attached to the carbon atom linked to the benzene or pyridine ring, especially allyl and 3-butenyl, or alkynyl, which comprises treating a compound of the formula (II):

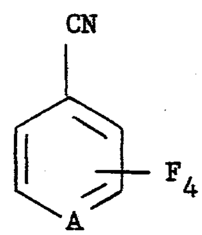

(II)

in which A has the meaning already given, with a Grignard reagent, RMgX, in which X is halo, under conditions suitable for the conduct of a Grignard reaction.

Conveniently, the Grignard reagent prepared in ethereal medium is added with stirring to compound II, also in ethereal medium, or vice versa. Reaction will usually take place in a temperature range of from -50°C to 100°C, preferably -25°C to 30°C and, more preferably, -25°C to 0°C. Typically, one ingredient is added to the other with stirring over a few hours and the reaction mixture allowed to stand for several minutes before the product is isolated. It may, however, be desirable to heat the reaction mixture after or during addition of the ingredients; for example, the mixture may be heated under reflux which, if tetrahydrofuran is the ether used, will be at a temperature of about 65°C.

Generally, the conditions of reaction will be those well known to be suitable for the conduct of Grignard reactions, it being important to exclude water.

It has been found advantageous, with regard to increased yields and reduced usage of Grignard reagent, to have present a quaternary ammonium salt, in particular, tetrabutylammonium bromide.

To isolate the product, the reaction mixture is cooled, if necessary, poured into water or over ice, acidified and the compound (I) extracted with, for example, methylene chloride.

Approximately 1 to 2 mols, typically about 1.5 mols, of Grignard reagent will normally be used for each mol of compound (II). When compound (II) is tetrafluoroterephthalonitrile, higher amounts of Grignard reagent transform compound (II), as will be described later, into a Grignard compound which will be decomposed during isolation of compound (I) in the aqueous medium.

The Grignard reagent, RMgX, may be prepared by methods well known for the preparation of Grignard compounds. X may be chloro, bromo or iodo.

Tetrafluoroterephthalonitrile is a known compound which may be obtained by fluorinating the corresponding tetrachlorinated compound with potassium fluoride in a polar aprotic solvent. The tetrachlorinated compound may be prepared from commercially available tetrachloroterephthaloyl chloride by treatment with aqueous ammonia to form the diamide which can be dehydrated with phosphorus oxychloride.

2,3,5,6-Tetrafluoro-4-cyanopyridine is also a known compound (C.A. Registry No.16297-07-7) which may be obtained from pentafluoropyridine (J.Fluorine Chem. [1973] 3 (3-4), 275-83: CA 80 59813X).

In another aspect of the invention, the compound of formula (I), in which A is C-CN, is treated with a Grignard reagent, $R^1MgX^1$, in which $R^1$ and $X^1$ have the meanings hereinbefore defined for R and X, respectively, under conditions suitable for the conduct of a Grignard reaction, to form a Grignard compound of formula (III):

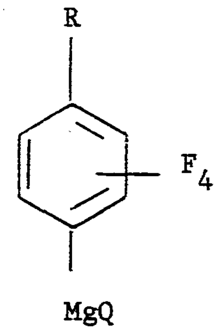

(III)

in which Q is CN or $X^1$.

The invention also includes the novel Grignard compound of formula (III). Q is cyano or halo.

The Grignard reagent $R^1MgX^1$ may be the same as or different from the Grignard reagent of RMgX used to prepare the compound of formula (I). As $R^1$ does not form part of the Grignard compound of formula (III), it may be chosen for reasons of cheapness and convenience. It is, however, possible to form the Grignard compound of formula (III) from tetrafluoroterephthalonitrile by reaction with normally more than 2 mols, for example, 3 mols/mol of the terephthalonitrile in a single-stage process, i.e. without isolation of the intermediate nitrile, in which case the Grignard reagents RMgX and $R^1MgX^1$ will be the same. This single-stage process forms yet another aspect of the present invention.

The Grignard compound of formula (III) may be used as a typical Grignard reagent in any of the reactions known to transform a Grignard compound, $ArMgX^2$, in which Ar is aryl and $X^2$ is halo, into a compound of $Ar-R^2$, in which $R^2$ is defined later. Its use as such forms still yet a further aspect of this invention.

Thus, the Grignard compound of formula (III) may be used to prepare compounds of the formula (IV):

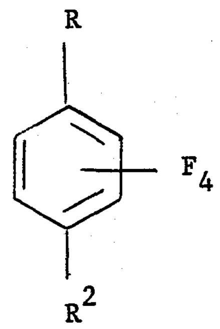

(IV)

in which R has the meaning hereinbefore defined, and $R^2$ is hydrogen, alkyl, particularly (C1-4) alkyl, especially methyl, alkenyl especially allyl, hydroxy, carboxy, a carboxylic ester, formyl, hydroxyalkyl especially hydroxyethyl, alkoxyalkyl, especially alkoxymethyl, alkylcarbonyl, or

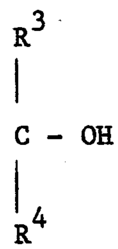

$R^3$ and $R^4$, which may be the same or different, being hydrogen or alkyl, particularly (C1-4) alkyl.

The table below illustrates with what substances the Grignard compound (III) may be reacted, under well known reaction conditions, to obtain the proposed values of $R^2$.

| Reaction of the Grignard compound of formula (III) with | $R^2$ |
|---|---|
| water or any other compound containing a Zerewitinoff active hydrogen atom | hydrogen |
| an alkyltosylate | alkyl |
| allyl iodide | allyl |
| (i) dry oxygen<br>(ii) dilute acid | hydroxy |
| (i) solid carbon dioxide<br>(ii) dilute acid | carboxy |
| ethyl chloroformate | carboxylic ethyl ester |
| ethyl formate or ethyl orthoformate or a tertiary formamide | formyl |
| (i) ethylene oxide<br>(ii) dilute acid | hydroxyethyl |
| chloroether of the type alkyl-O.$CH_2$Cl | alkoxyalkyl |
| (i) an alkyl cyanide (except acetonitrile)<br>(ii) dilute acid or, excess of an acyl chloride | alkylcarbonyl |
| (i) an aldehyde ($R^3$CHO)<br>(ii) dilute acid | $R^3$<br>- CHOH |
| and, when $R^3$ is H,<br>(i) formaldehyde or paraformaldehyde<br>(ii) dilute acid | - $CH_2$OH |
| (i) a ketone ($R^3$.CO.$R^4$)<br>(ii) dilute acid | $R^3$<br>- C - OH<br>$R^4$ |

In particular, a compound of formula (V):

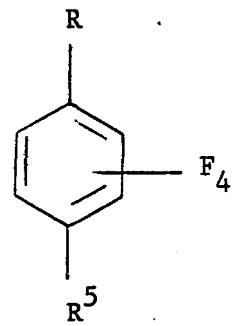

(V)

in which R has the meaning already given and $R^5$ is hydrogen or $(C_{1-4})$ alkyl, especially methyl, is prepared by contacting Grignard compound (III) with water (if $R^5$ is hydrogen) or an alkylating agent (if $R^5$ is alkyl). Conveniently, compound (V) may be prepared from tetrafluoroterephthalonitrile by a single-stage process, in which tetrafluoroterephthalonitrile is reacted with more than 2 mols/mol, for example 3 mols/mol, of a Grignard reagent RMgX under conditions suitable for the conduct of Grignard reactions and contacting the reaction product thereby obtained with water to give compound (V) in which $R^5$ is H or with an alkylating agent to give compound (V) in which $R^5$ is $(C_{1-4})$ alkyl.

Other compounds of formula (IV) may similarly be prepared by single-stage processes and such processes form still yet a further aspect of the present invention.

The compounds (I), (III), (IV) and (V) find use, for example, as chemical intermediates in the synthesis of pesticidal compounds.

The invention is illustrated by the following Examples 1 to 12 in which percentages are by weight.

Example 1

Preparation of 1-cyano-4-ethyl-2,3,5,6-tetrafluorobenzene

Tetrafluoroterephthalonitrile (0.01 mol) in tetrahydrofuran (10 ml) was added with stirring to ethylmagnesium bromide (0.01 mol) in tetrahydrofuran (10 ml) at 20°C. The resulting mixture, which rapidly darkened, was stirred and heated under reflux for 4 hours and then

allowed to cool to 20°C. The cooled mixture was poured over ice, acidified and extracted with methylene chloride. Glc (E301/150°)-mass spectrometry showed the presence of unreacted tetrafluoroterephthalonitrile (ca. 65%) together with 1-cyano-4-ethyl-2,3,5,6-tetrafluorobenzene (ca. 35%) m/e 188,203.

Traces only of dicyanoethyltrifluorobenzene, a cyanodiethyltrifluorobenzene and a tetraethyldifluorobenzene were detected.

Example 2

Preparation of 1-ethyl-2,3,5,6-tetrafluorobenzene

Tetrafluoroterephthalonitrile (0.01 mol) in tetrahydrofuran (10 ml) was added with stirring to ethylmagnesium bromide (0.03 mol) in tetrahydrofuran (20 ml). The resulting mixture was stirred and heated under reflux for 4 hours and then cooled to ambient temperature. The cooled mixture was poured over ice, acidifed and extracted with methylene chloride. Glc (E301/135°)-mass spectrometry showed that all but a trace of tetrafluoroterephthalonitrile had been consumed. One glc peak predominated which can be attributed to 1-ethyl-2,3,5,6-tetrafluorobenzene m/e 178,163. This product indicates that, before hydrolysis, the reaction mixture contained 4-ethyl-2,3,5,6-tetrafluorophenyl magnesium cyanide or bromide, probably, initially, the cyanide.

Example 3

Preparation of 1,4-dimethyl-2,3,5,6-tetrafluorobenzene

Methylbromide (5.7g, 0.06 mol) in tetrahydrofuran (20 ml) was added dropwise, under a $CO_2$ condenser, to magnesium (1.46g, 0.06 mol) in tetrahydrofuran (40 ml) stirred in the presence of a little iodine. There was a mild exotherm. The mixture was stirred for half an hour when nearly all the magnesium was consumed.

The mixture was cooled to -10°C and tetrafluoroterephthalonitrile (4.0g, 0.02 mol) suspended in tetrahydrofuran (20 ml) was added in portions while the temperature of the mixture which was a dark blue-green suspension, was kept at -10°C. The mixture was allowed to warm to room temperature and then added to dimethyl sulphate (30 ml) and left over a weekend. The mixture was

poured into water, acidified, extracted into methylene chloride and examined by glc (E301). A peak having the same retention time as an authentic sample of 1,4-dimethyl-2,3,5,6-tetrafluorobenzene was present. This structure was confirmed by mass-spectrometry/glc:m/e 178, 177, 163.

The yield of 1,4-dimethyl-2,3,5,6-tetrafluorobenzene was 0.0024 mol (12%).

Example 4

A slurry of tetrafluoroterephthalonitrile (100g; 0.5 mol) in tetrahydrofuran (2l) was stirred and cooled to -20°C. A solution of methylmagnesium bromide in tetrahydrofuran (260g; ca. 0.5 mol) was added at -20°C over 30 minutes. Analysis of a sample by glc showed the presence of 1-cyano-4-methyl-2,3,5,6-tetrafluorobenzene and tetrafluoroterephthalonitrile in the ratio of 4:3.

Two more lots of methylmagnesium bromide solution were added (each 130g; ca. 0.25 mol). Analysis showed that the 2 mols (approximately) of methylmagnesium bromide added was sufficient to consume nearly all the starting material..

Water (200ml) was added and the temperature of the reaction mixture allowed to rise to ambient. The mixture was rotary evaporated under reduced pressure to remove most of the tetrahydrofuran, treated with 4N HCl and extracted into methylene dichloride (total 950ml). Analysis by glc indicated that, on the assumption that all glc peaks derived from compounds having the same glc responses (per mol) as tetrafluoroterephthalonitrile, the mixture contained 1-cyano-4-methyl-2,3,5,6-tetrafluorobenzene (0.2455 mol, 49% yield) and 1,4-dicyano-2-methyltrifluorobenzene (0.0435 mol, 9% yield). No significant amount of starting material remained.

The methylene dichloride solution was rotary evaporated to leave a residue (89.7g) which was then distilled under reduced pressure (water pump) at 100 to 105°C. The distillate (43.7g) was shown by glc analysis to consist of 86% 1-cyano-4-methyl-2,3,5,6-tetrafluorobenzene (37.6g, 0.2 mol, 40% yield) and 11% of 1,4-dicyano-2-methyltrifluorobenzene. (More material distilled over after the still head temperature reached 105°C). A compound (28.7g), confirmed by nmr

analysis to be 1-cyano-4-methyl-2,3,5,6-tetrafluorobenzene (98% strength by glc), crystallised from the distillate on standing over a weekend. Analysis by glc showed the mother liquor to contain
80.8% 1-cyano-4-methyl-2,3,5,6-tetrafluorobenzene (m/e 188-189, 170, 139) and 15.9% of 1,4-dicyano-2-methyltrifluorobenzene (m/e 196, 195, 169).

Example 5

Preparation of 1-allyl-4-cyano-2,3,5,6-tetrafluorobenzene

Allylmagnesium chloride Grignard reagent

A small amount of neat allyl chloride (about ½g) was added to a mixture of magnesium (14.6g), a crystal of iodine and tetrahydrofuran (60ml) stirred under an atmosphere of nitrogen, when reaction was rapidly initiated. Allyl chloride (31.6g) in tetrahydrofuran (80ml) was then added at -10°C over 3 hours. The resulting allylmagnesium chloride was allowed to stand and assayed by both propylene evolution and by addition to an excess of 1N HCl and back titration with 1N NaOH.

Allylmagnesium chloride (10m.mol), prepared as described above, was added during 3 hours to tetrafluoroterephthalonitrile (1g, 5m.mol) in tetrahydrofuran (70ml) at -50°C. A blue colour formed early during the addition but, after work-up in a manner similar to that described in Example 1 (aqueous dilution; acidification; solvent extraction), samples for analysis were almost colourless. Essentially all of the Grignard reagent was consumed (no gas evolution during work-up) as was about 70% of the starting material. Little 1-cyano-2,3,5,6-tetrafluorobenzene was present. About 5% yield of a volatile component was detected. The addition of more Grignard reagent degraded the remaining dinitrile, but no more of the volatile component was formed.

Analysis by glc/mass spectroscopy showed the volatile component to have the formula

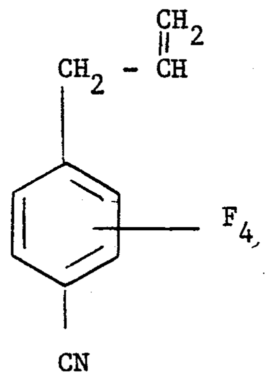

m/e 215, 188, 196

Preparation of 1-cyano-4-methyltetrafluorobenzene with and without tetrabutylammonium bromide present

Example 6

At -20°C

(a) A solution of methylmagnesium bromide in tetrahydrofuran (16m.mol, 12g diluted to 25ml) was added dropwise over 5 hours to tetrafluoroterephthalonitrile (2g, 10m.mol) stirred in tetrahydrofuran (20ml) at -20°C. The reaction mixture was left for 20 minutes after the Grignard reagent addition and then water added - methane evolution was measured - followed by 4N $H_2SO_4$. The products were extracted into toluene and analysed by glc. Results are given in Table 1.

(b) The procedure of Example 6(a) was repeated except that tetrabutyl ammonium bromide (1g) was added to the tetrafluoroterephthalonitrile/tetrahydrofuran mixture. This gave a yellow solution at 25°C and a white precipitate on cooling. On work-up more resin was apparent than in most other reactions at -20°C. Results are given in Table 1.

Unreacted methylmagnesium bromide was assayed by methane evolution, being 4.9 m.mol in 6(a) and 6m.mol in 6(b). Thus, in 6(b), only 10m.mol of Grignard reagent was consumed.

Table 1

| Example | Product | (m.mol) | Yield (%) [Conversion] |
|---|---|---|---|
| 6(a) | $CH_3C_6F_4CN$ | 5.00 | 50 |
| | $C_6F_4(CN)_2$ | 0.20 | [98] |
| | $CH_3C_6F_3(CN)_2$ | 0.91 | 9 |
| 6(b) | $CH_3C_6F_4CN$ | 6.74 | 67 |
| | $C_6F_4(CN)_2$ | 0.13 | [99] |
| | $CH_3C_6F_3(CN)_2$ | 1.0 | 10 |

These results show that the presence of tetrabutylammonium bromide leads to an increased yield of the desired product with an accompanying reduction in consumption of Grignard reagent.

Example 7

At 27°C

(a) A solution of methylmagnesium bromide in tetrahydrofuran (16m.mol, 25ml) was added over 3 hours to tetrafluoroterephthalonitrile (2g, 10m.mol) in tetrahydrofuran (20ml) at 27°C. Water was added to the purple reaction mixture about 20 minutes after completion of the Grignard addition. No methane was evolved. The mixture was acidified and the products extracted with toluene. Glc analysis of resulting highly coloured solution showed that all the tetrafluoroterephthalonitrile had been consumed. Results are given in Table 2.

(b) The procedure of Example 7(a) was repeated except that tetrabutylammonium bromide (1g) was added to the tetrafluoro-terephthalonitrile/tetrahydrofuran mixture giving a yellow solution. The reaction mixture after the Grignard addition was dark blue. Similar observations were made with respect to methane evolution and consumption of the dinitrile starting material. Results are given in Table 2.

Table 2

| Example | Product | (m.mol) | Yield (%) |
|---|---|---|---|
| 7(a) | $HC_6F_4CN$ | 0.25 | 2.5 |
| | $CH_3C_6F_4CN$ | 0.89 | 9 |
| 7(b) | $CH_3C_6F_4CN$ | 2.3 | 23 |
| | $CH_3C_6F_3(CN)_2$ | 0.49 | 5 |

These results show that the presence of tetrabutylammonium bromide leads to an increased yield of the desired product while surpressing the formation of 1-cyano-2,3,5,6-tetrafluorobenzene.

Example 8

Preparation of 1-(3-butenyl)-4-cyano-2,3,5,6-tetrafluorobenzene

3-butenylmagnesium bromide Grignard reagent

3-Butenyl bromide (13.2g, 10ml, 0.1mol) in tetrahydrofuran (20ml) was added to magnesium (3g) and a crystal of iodine in tetrahydrofuran (20ml). Initiation of reaction was indicated by a rapid exotherm. The reaction mixture was diluted to 80ml.

Part of the Grignard reagent prepared as above (60ml equivalent to 0.75mol of 3-butenyl bromide) was sufficient to consume 8.5g (0.0425mol) of tetrafluoroterephthalonitrile at -20°C in tetrahydrofuran. No significant quantities of gas were evolved on aqueous work-up.

The organic products were extracted into toluene and the solution rotary evaporated to leave 11.55g of residue. Of this, 10.6g was fractionally distilled under vacuum (20mm) to give a control portion (7.5g) which had a major component (73%) identified by glc/mass spectroscopy as $H_2C{=}CHCH_2CH_2C_6F_4CN$ (I), and a minor component (10%) thought to be $H_2C{=}CHCH_2CH_2C_6F_3(CN)_2$.

The yield of (I) was about 60% based on the dinitrile starting material.

Mass spec. data

m/e 229 (parent ion), 188 and 41 - major ions.
214, 209, 201 - minor ions.

Examples 9 to 11

Preparation of 1-benzyl, 1-n-propyl and 1-iso-propyl-4-cyano-2,3,5,6-tetrafluorobenzenes

The Grignard reagents listed in Table 3 were each (15m.mol) reacted with tetrafluoroterephthalonitrile (10m.mol) in tetrahydrofuran at -20°C. The products obtained (m.mol) are given together with the mass ion of the desired product.

Table 3

| Example No. | Grignard reagent | Products (m.mol)/mass ion |
|---|---|---|
| 9 | 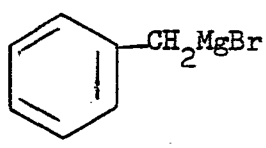 | $C_6F_4(CN)_2$ (5.0); 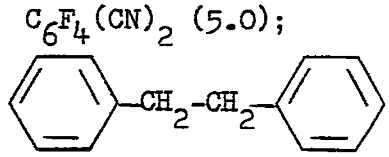 (5.0); 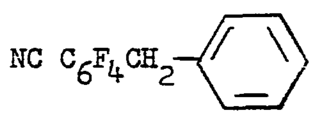 (4.0) m/e 265. |
| 10 | $CH_3CH_2CH_2MgBr$ | $C_6F_4(CN)_2$ (0.7); $CH_3CH_2CH_2C_6F_4CN$ (7.0) m/e 217. |
| 11 | $CH_3-CH(CH_3)MgBr$ | $C_6F_4(CN)_2$ (0.4); $CH_3-CH(CH_3)-C_6F_4CN$ (5.0) m/e 217. |

Example 12

Methylmagnesium bromide (10m.mol) was reacted with 2,3,5,6-tetrafluoro-4-cyanopyridine (10m.mol) in tetrahydrofuran at -20°C. The following products were obtained after work-up in the usual way.

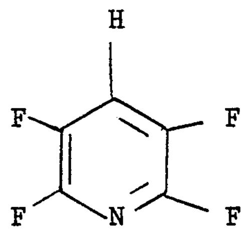

(1.3m.mol) m/e 151; presumably formed from

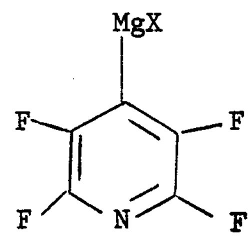

(X = CN/Br)

on addition of water during work-up.

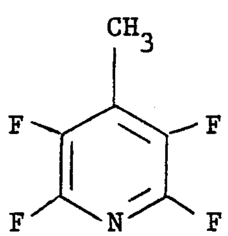

(0.7m.mol) m/e 165, 164.

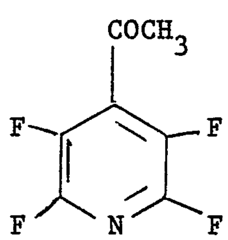

(0.2m.mol) m/e; the product to be expected from a Grignard reaction of this type.

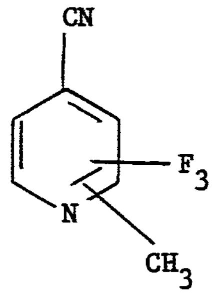

(0.2m.mol) m/e 172.

and unreacted starting product,

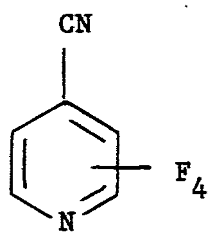

(1.6m.mol) m/e 176.

## CLAIMS

1. A process for the preparation of a compound of the formula (I):

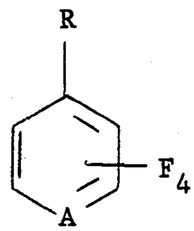

(I)

in which A is C-CN or N, R is alkyl, aralkyl, ($C_3$ or more) alkenyl, whose double bond is not attached to the carbon atom linked to the benzene or pyridine ring, or alkynyl, which comprises treating a compound of the formula (II):

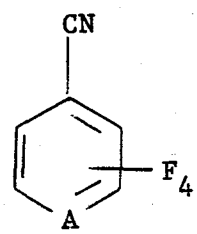

(II)

in which A has the meaning already given, with a Grignard reagent, RMgX, in which X is halo, under conditions suitable for the conduct of Grignard reactions.

2. A process according to claim 1 in which there is used from 1 to 2 mols of the Grignard reagent, RMgX, for each mol of compound (I).

3. A process for the preparation of a Grignard compound of the formula (III):

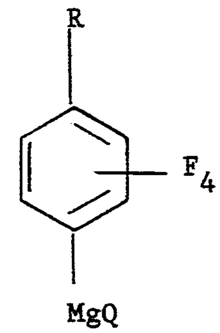

(III)

in which Q is CN or $X^1$, $X^1$ is halo and R has the meaning given in claim 1, which comprises treating the compound (II) of claim 1 in which A is C-CN with a Grignard reagent, $R^1MgX^1$, in which $R^1$ has the meaning given to R but may be the same as or different from R, under conditions suitable for the conduct of Grignard reactions.

4. A process for the preparation of the compound (III) of claim 3 which comprises treating the compound (I) of claim 1 with more than 2 mols of the Grignard reagent RMgX of claim 1 for each mol of compound (I), under conditions suitable for the conduct of Grignard reactions.

5. The compound (III) of claim 3.

6. A Grignard compound obtained by the process of claim 3 or 4 which contains the residue:

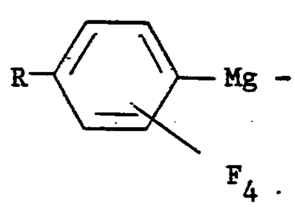

7. The use of the Grignard compound (III) of claim 3 or 6 to prepare a compound of the formula (IV):

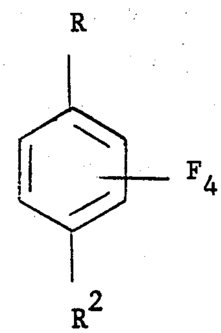

(IV)

in which R has the meaning given in claim 1 and $R^2$ is hydrogen, alkyl, alkenyl, hydroxy, carboxy, a carboxylic ester, formyl, hydroxyalkyl, alkoxyalkyl, or

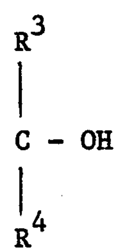

in which $R^3$ and $R^4$, which may be the same or different, are hydrogen or alkyl, by a Grignard reaction conventionally used to transform a Grignard compound, $ArMgX^2$, in which Ar is aryl and $X^2$ is halo, into a compound $Ar-R^2$.

8. A process for the preparation of a compound (V):

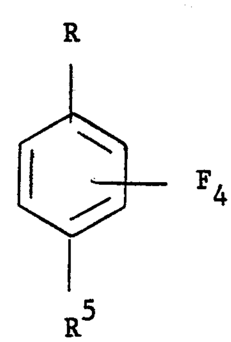

(V)

in which $R^5$ is hydrogen or $(C_{1-4})$ alkyl which comprises contacting the compound (III) of claim 3 or 6 with water or an alkylating agent.

9. A process for the preparation of the compound (V) of claim 8 which comprises reacting tetrafluoroterephthalonitrile with more than 2 mols of the Grignard reagent RMgX of claim 1 for each mol of tetrafluoroterephthalonitrile under conditions suitable for the conduct of Grignard reactions and contacting the reaction product thereby obtained with water or an alkylating agent.

MJH/BH

26.1.84.

0119701

European Patent Office

# EUROPEAN SEARCH REPORT

Application number

EP 84 30 0653

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | FR-A-2 132 244 (K. THOMAE) * Page 1; pages 4,5, example 1 * | 1-8 | C 07 B 17/00<br>C 07 D 213/61<br>C 07 C 17/26<br>C 07 C 121/60<br>C 07 C 120/00 |
| Y | TETRAHEDRON LETTERS, vol. 22, no. 16, 1981, pages 1509-1510, Pergamon Press Ltd., GB G.P. AXIOTIS: "Reaction of organometallic reagents with triethoxyacetonitrile. A new and short synthesis of alpha-ketoesters" * Page 1509, lines 1-4 * | 1-8 | |
| Y | JOURNAL OF THE CHEMICAL SOCIETY, (C), 1967, pages 2091-2095 R.E. BANKS et al.: "Heterocyclic polyfluoro-compounds. Part XII. Synthesis and some reactions of 2,3,5,6-tetrafluoro-4-iodopyridine" * Page 1967, scheme 2 * | 1-8 | TECHNICAL FIELDS SEARCHED (Int. Cl. 3) |
| Y | TETRAHEDRON LETTERS, vol. 21, pages 845-848, 1980, Pergamon Press Ltd., GB A. MINATO et al.: "Selective mono-alkylation and arylation of aromatic dihalides by palladium-catalyzed cross-coupling with the Grignard and organozinc reagents" * Page 846 * | 1-8 | C 07 D 213/00<br>C 07 C 17/00<br>C 07 C 120/00<br>C 07 C 121/00 |

-----

The present search report has been drawn up for all claims

| Place of search | Date of completion of the search | Examiner |
|---|---|---|
| THE HAGUE | 01-06-1984 | VERHULST W. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503. 03.82